Europäisches Patentamt

European Patent Office

Office européen des brevets

(11) Publication number: **0 233 177**
**B1**

## EUROPEAN PATENT SPECIFICATION

(45) Date of publication of patent specification: **25.01.89**

(51) Int. Cl.⁴: **C 12 Q 1/68,** G 01 N 33/53

(21) Application number: **85904157.6**

(22) Date of filing: **20.08.85**

(86) International application number:
**PCT/NO85/00049**

(87) International publication number:
**WO 87/01134 26.02.87 Gazette 87/05**

(54) PHOTOIMMUNE DETECTION OF DNA AND RNA.

(43) Date of publication of application:
**26.08.87 Bulletin 87/35**

(45) Publication of the grant of the patent:
**25.01.89 Bulletin 89/04**

(84) Designated Contracting States:
**AT BE CH DE FR GB IT LI NL SE**

(56) References cited:
**EP-A-0 102 228
EP-A-0 131 830
GB-A-2 125 964**

**Chemical Abstracts, vol. 100 (1984), abstract
no. 19843g, Mutat. Ros. 1983, 112(5), 287-99**

**Chemical Abstracts, vol. 95 (1981), abstract no.
111 232h, Biochim Biophys Acta, 1981, 655(1),
54-60**

**"Derwent's Abstract No. 84-169757/27, B04, SU
653 273"**

**Chemical Abstracts, vol. 101 (1984), abstract
no. 206762n, Photochem Photobiol. 1984,
40(4), 465-71**

**Pharmacia "Molecular Biologicals" Catalog
(1984), pp. 135-139**

(73) Proprietor: **APOTHEKERNES LABORATORIUM
A.S.
P.O. Box 158
Skoyen, N-0212 Oslo 2 (NO)**

(72) Inventor: **EGGSET, Guri
Trymsvei 3
N-9000 Tromso (NO)**
Inventor: **GUDDAL, Per, Henrik
Sjotun
N-9100 Kvaloysletta (NO)**
Inventor: **KROKAN, Hans, Einar
Mellombrottet 11
N-3900 Porsgrunn (NO)**
Inventor: **LINDQVIST, Bjorn, Hadar
Ankerveien 13B
N-0390 Oslo (NO)**
Inventor: **VOLDEN, Gunnar
Kapteinveien 23
N-9014 Hapet (NO)**

(74) Representative: **Henningsson, Gunnar et al
Bergling & Sundbergh AB Box 7645
S-103 94 Stockholm (SE)**

(56) References cited:
**Chemical Abstracts, vol. 99 (1983), abstracts
no. 66 816w, Carcinogenesis (London) 1983,
4(6), 745-50**

## Description

Field of the Invention

The present invention relates to an assay method for detection of specific nucleotide sequences in DNA or RNA, and to kits useful for performing such assays.

Background of the Invention

Molecular hybridization is extremely useful in the detection of specific nucleotide sequences in genetic materials. Generally, single-stranded chromosomal DNA from a test specimen is denatured and attached to a DNA-binding membrane or filter support. The membrane-filter is brought into contact with a labeled single-stranded polynucleotide probe under conditions promoting hybridization of complementary DNA sequences. Double stranded "duplex" or "hybrid" molecules are detected by a variety of techniques, depending on the nature of the particular label used. In order for hybridization to occur, the probe-molecules must contain sequences substantially complementary to those sequences in the nucleic acid to be detected. An example of such a hybridization system is U.S. Patent No. 4,358,535 to Falkow et al.

Until recently, hybrid molecules have been detected exclusively with radioisotopically labeled probes. The presence of hybrids is detected by scintillation counting or autoradiography, thereby providing a quantitative assay for the presence of the specific DNA of interest. Although autoradiography is a sensitive method, it can be time consuming. Moreover, hybrid-detecting methods involving the use of radioisotopes must be performed under stringent safety precautions. Radioisotopes are extremely expensive, and in some cases have a limited shelf life due to rapid disintegration.

A currently used method for radioactively labeling DNA probe molecules incorporates $^{32}$p-, $^{14}$C- or $^{3}$H-containing nucleotides by the "nick translation" method of Rigby et al., J. Mol. Biol., $113$:237 (1977). According to this technique, nuclease treatment of the probe nucleic acid is necessary to nick or open gaps in one of the strands. A radiolabelled nucleotide is then inserted into the DNA with a polymerase.

A more recent innovation in molecular genetic probe technology involves utilization of the nick-translation technique to incorporate biotinylated dUTP into probe DNA.

The nick-translation technique requires nuclease treatment of the template nucleic acid to open gaps in one of the strands. A radiolabelled or biotin-substituted nucleotide is inserted with a polymerase. The duplex is then split with the thus-labelled strand being used as a probe.

Biotin-labeled DNA may be detected by a soluble complex of biotin-bound horseradish-peroxidase and streptavidin, or by means of a biotin-specific antibody, followed by a secondary fluorescein-labeled antibody.

A more recent technique dispensing with the need for nick-translation is described in European Patent Application Publication 128018. Nucleotides of the probe molecule are modified *in situ*, for example, by alkylation. Antibodies to the alkylated probe are then used to detect the formation of duplexes.

Yet another chemical-modification approach is disclosed in European Patent Application Publication 122614. A chemically-labeled nucleotide, e.g., a biotinylated nucleotide, capable of acting as a substrate for terminal deoxynucleotide transferase, is polymerized onto the terminal end of the probe molecule. A polymer with a biotin-containing analog of TTP is formed on the 3'-OH terminus of the probe molecule. Antibodies to the probe molecule may be used to detect the presence of hybrid molecules.

The above methods, relying on the insertion of radio-labeled or chemically-modified nucleotides, on chemical modification of probe DNA molecules, or on terminal polymerization of biotinylated nucleotides, are costly and time consuming. Although non-isotopic labelling of nucleic acids has been successfully employed in molecular hybridization and diagnostic tests, Langer et al., Proc. Natl. Acad. Sci. USA, $68$:6633 (1981), Singer et al., Proc. Natl. Acad. Sci. USA, $79$:7331—35 (1982), Hutchinson et al., J. Cell. Biol., $95$:609—618 (1982), the key substance, biotinylated dUTP, is a high-cost product because of its intricate chemical synthesis.

Antibodies to UV-irradiated DNA have been prepared by Mitchell et al., Biochem. Biophys. Acta, $655$:54—60 (1981) and Eggset et al., Carcinogenesis $4$:745—750 (1983). However, such antibodies have not heretofore been used in conjunction with molecular probes to detect the presence of specific nucleotide sequences.

What is needed is a simple but sensitive method for detecting DNA which dispenses with the need for time consuming and costly radiolabelling or intricate chemical synthesis of specialized nucleotides.

Summary of the Invention

A method for detecting the presence of specific nucleic acid in a sample is provided. A polynucleotide probe which will hybridize with the suspect nucleic acid is prepared. The probe is labelled by inducing the formation of UV-photo-products by exposure to ultraviolet radiation. The photo-products are recognizable by antibodies to the UV-labelled probe. The labelled probe is then contacted with single-stranded nucleic acid from the sample under hybridizing conditions. Hybrid complexes are detected with anti-UV-nucleic acid antibodies labelled with a signalling means. The antibodies bind the UV-labelled probe to indicate the presence of the suspect nucleic acid in the sample.

In one embodiment of the invention, the labelled probe includes a polypyrimidine nucleotide sequence or "tail" at the 3'-OH terminus. UV-photoproducts, principally pyrimidine dimers, are created in the tail by exposure to UV-radiation.

The tail is generated by cloning the probe directly into a polypyrimidine nucleotide sequence or by polymerizing pyrimidine nucleotides onto the 3'-OH terminal end of the probe with the enzyme terminal transferase.

The invention also relates to a kit for detecting the presence of specific nucleic acid in a suspect sample. The kit contains a supply of UV-labelled nucleic probe molecules containing UV-photoproducts. The probe is selected so as to hybridize with the nucleic acid to be detected. The kit further contains means for contacting the suspect sample with the UV-labelled probe to form hybrid complexes. The kit contains a supply of anti-UV-nucleic acid antibodies which bind the UV-labelled probe. The anti-UV-nucleic acid antibodies are labelled with a signalling means. The kit may contain a means for measuring the signal to indicate the presence of or extent of the nucleic acid being detected.

Yet another kit, which may be used to quantify total DNA in a test sample, includes a supply of anti-UV-DNA antibody, a panel of DNA-containing samples of known DNA concentrations as standards, a signalling means for indicating binding of anti-UV-DNA antibody, and means for comparing the level of the signal generated by DNA in the test sample with the levels of signal generated by the various members of the standard panel.

It is therefore an object of the invention to provide a method for detecting the presence of specific nucleic acids in a sample.

It is an object of the invention to provide a method for detecting specific nucleic acids without the need for radioactive labelling or chemical synthesis of biotinylated or other synthetic nucleotides.

It is an object of the invention to provide a method for detecting nucleic acids which may be used to detect the presence of organisms containing either single-stranded or double-stranded genetic material.

It is a further object of the invention to provide a test kit for the detection or quantification of specific nucleic acids.

It is an object of the invention to provide a kit for detecting the amount of total DNA in a test sample.

These and other objects are apparent from the following disclosure.

Brief Description of the Figures

Figue 1 contains chemical structures of UV-induced DNA-photoproducts which may be generated in probe molecules according to the present invention.

Figure 2 is a schematic sequence illustrating the process of the present invention for photoimmune detection of DNA wherein a single-stranded DNA-probe has been extended with a poly-dT tail sequence before UV-irradiation to increase the number of UV-photoproducts for antibody binding.

Figure 3 is a photograph of the hybridization of a representative UV-irradiated DNA probe to homologous DNA which is attached to a membrane filter. Hybrids were detected with rabbit anti-UV-DNA antibody and biotinylated goat-anti-rabbit IgG, followed by incubation with streptavidin and biotinylated alkaline phosphatase.

Figure 4 is a photograph of the hybridization of a UV-irradiated bacteriophage Hyl7 DNA-probe to different concentrations of single-stranded Hyl7 DNA bound to a filter membrane in the presence of the negative controls bacteriophage lambda-DNA and calf-thymus-DNA.

Figure 5A is a photograph of a conventional ethidium bromide staining of restriction enzyme-digested DNA following agarose gel electrophoresis.

Figure 5B is a photograph of the same DNA fragments following alkaline denaturation, transfer to a filter-membrane by electroblotting, and incubation with anti-UV-DNA-antibody conjugated to horseradish peroxidase.

Detailed Description of the Invention

By "UV-photoproduct" as used herein is meant a nucleotide sequence which has undergone chemical modification in one or more nucleotides by exposure to ultraviolet radiation.

By "UV-nucleic acid" or "UV-DNA" is meant nucleic acid or DNA which has been exposed to ultraviolet radiation thereby causing the formation of UV-photoproducts.

By "anti-UV-nucleic acid antibody" or "anti-UV-DNA antibody" is meant antibodies which bind UV-nucleic acid or UV-DNA but not normal nucleic acid or normal DNA.

The present invention is based upon a form of in situ-labelling of DNA by means of UV-light which occurs in a one-step process, as distinguished from the incorporation of radiolabelled or chemically-labelled nucleotides using the nick-translation method, or the terminal polymerization of such labelled nucleotides to probe molecules.

A polynucleotide probe molecule is prepared which contains a nucleotide sequence substantially complementary to the nucleotide sequence of the unknown genetic material to be detected. The probe comprises a single strand, or partially denatured double strand, of nucleic acid. The probe may be DNA or RNA, which is extracted from the relevant genetic material and cloned or synthesized chemically when the nucleotide sequence is known. According to U.S. 4,358,535, probe molecules may be obtained from messenger RNA, from cDNA generated by reverse transcription of messenger RNA, or from cleavage of the genome, followed by cloning of the probe molecule in accordance with existing techniques, e.g. Maniatis, T., et al., *Molecular Cloning: A Laboratory Manual,* Cold Spring Harbor Laboratory (1982), p. 187, 211.

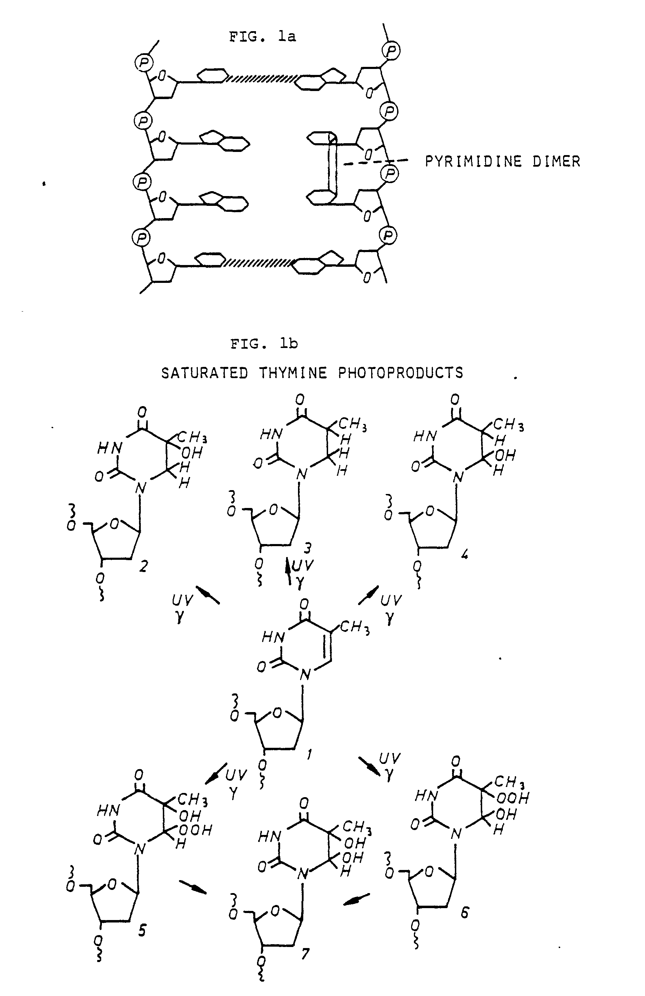

The DNA-probe molecules are irradiated with a relatively low dose of ultraviolet radiation of between 200—400nm wavelength, for example, 2500 J/m² at 254nm, in order to form UV-photoproducts. Most of the photoproducts thus formed are pyrimidine dimers, but a few saturated pyrimidine products also occur. See Figure 1. Binding occurs between the C-5 position in a first pyrimidine and the C-5 position in a neighboring pyrimidine base, and between the C-6 position in the first pyrimidine base and the C-6 position in the neighboring pyrimidine base. Binding may also occur between the C-6 position of the pyrimidine base closest to the 3'-OH terminal end of the probe and the C-4 position of the neighboring pyrimidine base. Irradiation can also include saturation of the double bond between the C-5 and C-6 position of any pyrimidine ring through UV-induced uptake of hydrogen and/or hydroxyl. The DNA probe molecules bearing the aforementioned photoproducts are potent antigens to which antibodies may be raised.

Photoproducts, can, as an alternative, be produced by UV-light in combination with a photoreactive chemical compound such as furocoumarin.

The sample material to be assayed comprises nucleic acid, either single or double stranded. When assaying for nucleic acid form organisms containing double-stranded nucleic acids, a denaturing step is required. Denaturing techniques are well-known in the art and do not form part of the present invention. See, for example, Maniatis, T., et al., *Molecular Cloning: A Laboratory manual*, Cold Spring Harbor Laboratory (1982), p. 314, 321, 383; Gergen et al., Nucleic Acids Res. *7*: 2115—2136 (1980). Heat denaturation at 95—100°C for five minutes, followed by rapid chilling is the preferred method of denaturation.

The irradiated probe-DNA is incubated with the sample nucleic acid material containing the purported nucleotide sequence. According to one technique, the sample nucleic acid is affixed to a support material capable of binding nucleic acid. Cellulosic supports are preferred, particularly nitrocellulosic filter papers, which readily bind DNA. One such material is the "Gene Screen" hybridization transfer membrane available from New England Nuclear. It should be understood, however, that attachment to a solid support material is merely one way of visualizing hybridization, it being contemplated that the hybridization process may also be performed in solution.

Hybridization *per se* is a well-known technique and is described in the literature. See, for example, *Molecular Cloning, supra*; U.S. 4,358,535. The particular hybridization technique utilized will depend upon the nature of the sample material.

UV-photoproducts in DNA do not decrease hybridization capacity significantly when the probes are greater than 100 bases. Smaller probes will also hybridize well with a low UV-dose.

The formation of hybrid complexes between the probe-nucleic acid and nucleic acid in the test specimen is detected by anti-UV-nucleic acid antibodies which are labelled with a suitable signal means. Anti-UV-nucleic acid antibodies may be produced according to Mitchell, D. L. and Clarkson, J. M., Biochem. Biophys. Acta, *655*: 54—60 (1981), Wani, A. A. et al., Photochemistry and Photobiology, *40* (4): 465—471 (1984) or Eggset et al., Carcinogenesis *4*:745—750 (1983), which disclose immunization of rabbits with single-stranded UV-irradiated calf thymus DNA conjugated to methylated bovine serum albumin. Antibodies with specificity against un-irradiated DNA may be removed by affinity column chromatography through cyanogen bromide activated Sepharose® (Pharmacia, Sweden) coupled to un-irradiated DNA (Eggset et al., Supra). We have found that the unbound antibody fraction specifically binds UV-irradiated DNA with ten times greater affinity against single-stranded DNA compared to double-stranded DNA.

Polyclonal antibodies against UV-nucleic acid prepared in this manner, or specific monoclonal antibodies thereto, may be used to detect molecular hybridization according to the present invention.

The antibody is labelled with a signalling means. The signal is detected or measured as an indication of the presence or extent of the suspect nucleic acid in the sample. Suitable signalling means used to label the anti-UV-nucleic acid antibodies include chemical labels which are detected on the basis of their own physical properties. Such chemical labels comprise, by way of illustration, and not by way of limitation, color-indicating compounds, particularly fluorescent dyes such as fluorescein and rhodamine, or reagents of high electron density such as ferritin, hemocyanin and colloidal gold. Alternatively, the signalling means may comprise a substance which is detected by its binding or reactive properties, such as an enzyme which catalyzes a reaction leading to the generation of detectable reaction products. Examples of such enzymes comprise peroxidase and alkaline phosphatase.

Most advantageously, a secondary antibody which binds to the anti-UV-nucleic acid antibodies is labelled with the signalling means. For example, where the anti-UV-antibodies are raised from rabbits, the secondary antibody may comprise swine-anti-rabbit IgG, conjugated to horseradish-peroxidase. By the addition of suitable substrates, in this case hydrogen peroxide and diaminobenzidine (DAB), the enzyme will give a color reaction.

The secondary antibody may comprise a biotinylated antibody which binds the anti-UV-nucleic acid antibody and further binds streptavidin, which may in turn be coupled to a signalling means.

The anti-nucleic acid antibodies may be added to the system following a suitable incubation period to allow the probe molecules to hybridize. Alternatively, the antibodies may be permitted to bind to the probe prior to hybridization.

In one embodiment of the invention, a polynucleotide probe is prepared which includes a polypyrimidine nucleotide sequence at the 3'-OH terminus of the probe to increase the sensitivity of the assay. Poly-dT is the preferred pyrimidine nucleotide. Polypyrimidine nucleotide "tail" sequences may be thus generated by cloning the probe directly into a polypyrimidine sequence. The probe is inserted into a cloning vector adjacent to a poly-dT sequence. Useful cloning vectors for this purpose include single-stranded phage vectors such as M-13 which are able to secrete phage particles containing poly dT-tailed DNA probe molecules in single-stranded form, ready for use.

The tail sequence may also be generated by enzymatically polymerizing pyrimidine nucleotides onto the 3'-OH terminal end of the probe with terminal deoxynucleotide transferase. A typical tail prepared in this manner comprises between about 600 and about 2000 nucleotides.

The creation of polypyrimidine nucleotide tails on the probe molecules results in increased sensitivity in the assay technique. Probes with poly-dT tails constitute a larger target for generation of UV-photoproducts. Moreover, after the probe molecules have been hybridized to form duplexes with complementary DNA, the single-stranded poly-dT tails remain unhybridized, and protrude from the duplex molecules. The photoproducts contained in the tail are easily accessible to anti-UV-DNA antibodies, and provide improved antibody binding, making the test system more sensitive.

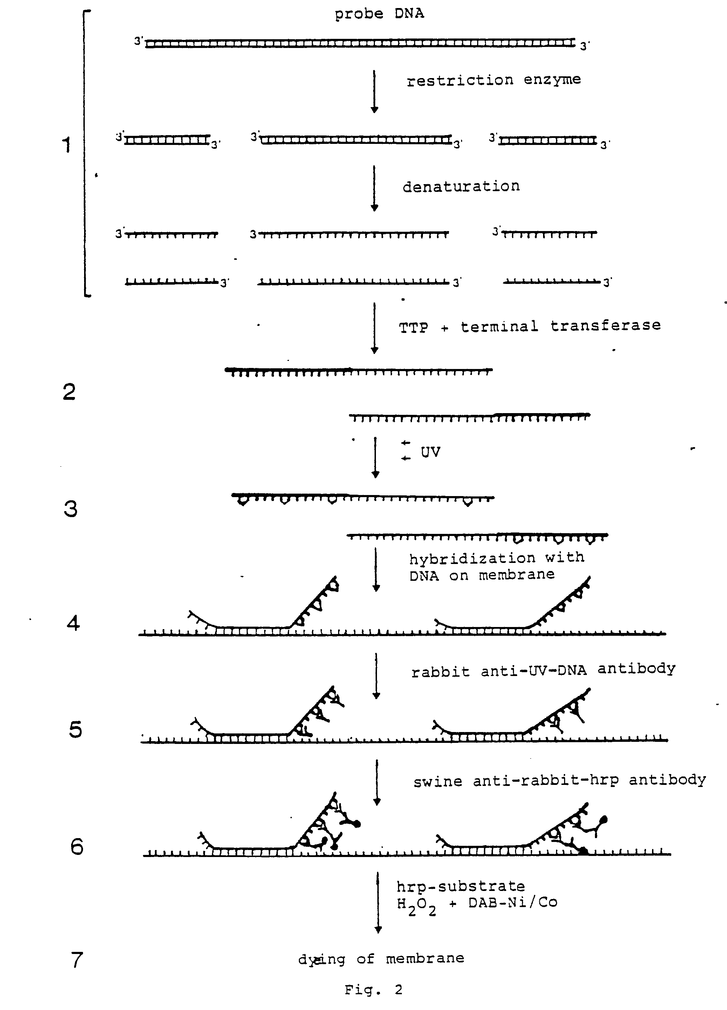

The present process of photoimmune detection of hybrid molecules is illustrated diagrammatically in Figure 2:

(1) Preparation of probe-DNA: The DNA containing the desired probe sequences are cleaved with restriction enzymes or fragmented mechanically to create more ends. The probe-DNA is then denatured at 100°C for five minutes, followed by cooling on ice (Fig. 2—1).

(2) (Optional) The probe-DNA is treated with terminal transferase and dTTP to generate a poly-dT-tail sequence at the 3'-OH end of the molecule (Fig. 2—2). Alternatively, the tail is put into position by cloning the probe directly into a poly-dT-sequence.

(3) The probe-DNA is labelled by UV-irradiation (254 nm) which leads to the formation of UV-protoproducts that are recognizable by antibodies (Fig. 2—3).

(4) DNA-DNA- (or DNA-RNA-) hybridization: The labelled probe is incubated with a membrane containing samples of single-stranded DNA (or RNA), followed by washing (Fig. 2—4).

(5) The membrane with the hybridized probe-DNA is incubated with anti-UV-DNA-antibody (Fig. 2—5). Excess antibody is washed off.

(6) The complex thus formed is incubated with secondary antibody to the anti-UV-DNA-antibody, e.g. swine-anti-rabbit IgG, linked to horseradish-peroxidase (Fig. 2—6). Excess antibody is washed off.

(7) The membrane is developed by means of hydrogen peroxide and diaminobenzidine (Fig. 2—7).

Detection and/or quantification of total nucleic acid or total DNA according to the present method may be used in recombinant DNA technology, such as in cDNA cloning, where the amount of genetic material to be assayed is often too small for conventional agarose/ethidium bromide visualization. The method may also be used to monitor the level of nucleic acid during the production of vaccines, hormones, monoclonal antibodies, and other pharmacological substances produced for *in vivo* administration. There exists a great need for demonstrating the absence of nucleic acid in such preparations, owing to the possible presence of oncogenes in contaminating nucleic acid. The present photoimmune detection system offers an easy, inexpensive and sensitive method for achieving this goal.

A kit for quantifying total nucleic acid in a sample includes a supply of anti-nucleic acid antibody, a panel of nucleic acid-containing samples of known nucleic acid concentration as standards, signalling means for indicating the binding of anti-nucleic acid antibody, and a means for comparing the level of signal generated by nucleic acid in the test sample with the levels of signal generated by the various members of the standard panel. The signalling means may suitably comprise any of the various signalling means discussed above, for example, an enzyme such as horseradish-peroxidase conjugated to a swine-anti-rabbit IgG, and substrates for the enzyme providing a color producing reaction. The panel of standards may be distributed in wells surrounding the test sample on a nucleic acid-binding support. The nucleic acid in the sample is quantified by matching the color intensity of the developed sample with the appropriate standard.

The present method for detecting *specific* nucleic acid may likewise be adapted to kit form. Such a kit may contain a supply of UV-labelled nucleic acid probe molecules selected so as to hybridize with the nucleic acid to be detected. The kit further contains a means for contacting the suspect sample with the probe molecules and a supply of anti-UV-nucleic acid antibodies which bind to the UV-labelled probe. The kit contains a signalling means for indicating the binding of anti-UV-nucleic acid to the probe. The contacting means may comprise a support such as nitrocellulose paper onto which the sample nucleic acid is deposited.

The present method for photoimmune detection of specific nucleic acid may be used to demonstrate the presence of particular bacteria or other organisms; to determine the pressence of resistance and/or virulence-determinants in micro-organisms; to diagnose genetic disease; to perform chromosome karyotype analyses; and to perform gene diagnostic methods such as oncogene analysis, tissue-typing or determination of predisposition to disease. Each of the aforesaid procedures involves the detection and visualization of specific nucleotide sequences in genetic material under study.

The present method of photoimmune detection of nucleic acids has the following noteworthy advantages over detecting means utilizing radioisotopes:

(a) It is easy to label probes by UV irradiation. UV-lamps are standard equipment in most laboratories in the form of germicidal lamps. Even short probes, for instance, probes formed from synthetic DNA, may be utilized according to the present invention by first attaching a polypyrimidine nucleotide sequence to the 3'-OH probe terminus. Such terminal sequences may contain many photo-products which can be easily recognized by antibodies.

(b) No expensive chemicals or costly radioactive nucleotides are required for probe labelling.

(c) The UV-labelled probe molecules are stable. It is believed that such probe molecules may be stored for years after labelling. It is thus possible to generate and store extensive probe libraries for use according to the method of the present invention.

(d) The problems and dangers connected with handling of radioactive isotopes are avoided.

(e) The method is quicker than existing radioisotope-labelling methods, as no time is spent in developing audioradiographic films, or in scintillographic counting.

Photoimmune detection of nucleic acid has the following advantages over detection means utilizing biotinylated dUTP:

(a) Photoimmune detection, which relies on the generation of antigenic determinants *in situ* with UV-light, is much simpler and quicker than assay methods using nick-translation for the incorporation of biotinylated dUTP.

(b) The present method is more economical, as the use of expensive chemically-synthesized biotinylated dUTP is avoided.

(c) Labelling by nick-translation is unsuitable for extremely short probes, and is useless for probes formed from single-stranded nucleic acids. The present photoimmune detection method may be employed for all types of probes. Short probes may be used by first attaching a polypyrimidine nucleotide sequence to the 3'-OH end of the probe.

(d) The present method is at least as sensitive in detecting nucleotide sequences as the nick-translation biotin-labelling method.

(e) The present method may be used as a highly sensitive technique to detect the presence of total nucleic acid in a sample, whereas the prior art methods may be used to detect specific nucleic acid sequences only.

The present invention is illustrated through the following non-limiting examples.

### Example 1
### Photoimmune Detection of DNA
### Probes Hybridized to Sample DNA

Plasmid pPHR20 containing a 2.1 kb Kpnl-fragment of ribosomal DNA isolated from the slime mold *Physarum polycephalum* was used as a probe. The probe DNA was labelled by UV-irradiation (254 nm, approximately 4 kJ/m²), linearized by cleavage with the restriction enzyme Pstl and denatured by heat treatment (95°C, 5 min.), followed by a quick chill in ice water to keep the DNA single-stranded.

The DNA samples to be tested were generated as follows. The plasmid pPHR20 as cleaved into four fragments of the following amounts with the restriction enzyme Pstl: (1) 250 ng; (2) 680 ng; (3) 68 ng; and (4) 6.8 ng. A fifth sample comprised a 343 ng fragment of Kpn-I-cleaved pPHR20. A sixth sample comprised 100 ng of the plasmid pPYA101. The fragments were separated by electrophoresis on a 0.7% agarose gel. The gel was then soaked in alkaline solution (0.2 M NaOH, 5 min.), neutralized in electrophoresis buffer, pH 7.5, and transferred onto a New England Nuclear "Gene Screen" membrane by electroblotting (electrophoretic transfer of the DNA fragments out of the gel plane onto the attached membrane filter giving an "offprint" of the DNA fragment pattern on the membrane). The membrane was then baked in a vacuum oven for 2 hrs at 80°C. The UV-irradiated single-stranded DNA-probe was hybridized to the baked membrane at 42°C for 18 hours in 50% formamide according to Maniatis, et al., *Molecular Cloning*, p. 324—27. Following hybridization, nonspecific antibody binding sites were saturated by incubating the membrane with a 10% solution of swine serum in tris-buffered saline, TBS (20 mM Tris-HCl, pH 7.6, 0.5 M NaCl) at 20°C for one hour with gentle shaking. The primary antibody, rabbit-anti-UV-DNA antibody, in a 10% swine serum dilution, was subsequently allowed to attach to the membrane-bound DNA by incubation for 30 min at 20°C. After washing (3 × 5 min. in TBS, 0.05% Tween 20) the secondary antibody, biotinylated goat-anti-rabbit IgG was allowed to bind, followed by incubation with streptavidin and biotinylated alkaline phosphatase (protocol by the supplier — Bethesda Research Laboratory).

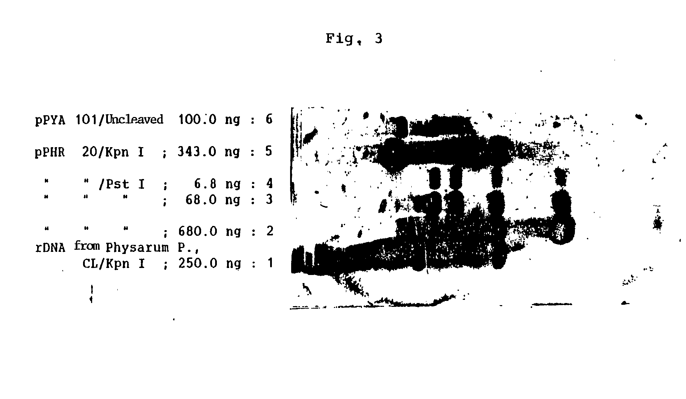

The results shown in Fig. 3 illustrate the sensitivity of the detection system. The amount of DNA loaded in lanes 1, 2 and 5 giving bands containing DNA in the 100 ng range (which is the normal level for ethidium bromide staining), appears as heavily overloaded when probed with the photoimmune detection system. However, DNA bands in the 0.1—10 ng range (lanes 3 and 4) are more appropriate for qualitative identification.

### Example 2
### Photoimmune Detection with DNA-
### Probes Extended with PolydT-tails

Photoimmune detection of specific DNA from the bacteriophage Hyl7 was demonstrated using DNA-probe molecules with appropriate poly-dT tail sequences. Hyl7 is an artificially-made bacteriophage consisting of a defined chromosome segment from each of the bacteriophages P2 and P4. Various quantities (8—1000 ng) of the DNA-samples were applied to a membrane (Gene-Screen Hybridization Transfer Membrane NEF—927, New England Nuclear) by means of a microfiltration apparatus (Bio-Dot Microfiltration Apparatus, Bio-Rad Laboratories) in accordance with the instruction manual for the "Gene-Screen" membrane (New England Nuclear Catalogue No. NEF 872, *Gene-Screen: Hybridization Transfer Membrane-Instruction Manual*). Single-stranded ("ss") and double-stranded ("ds") DNA from phage lambda and single-stranded calf thymus DNA ("C.th.DNA"), were used as negative controls.

A probe which consisted of Hyl7-DNA cleaved with the restriction enzyme HindIII was used for the hybridization-reaction. The fragments were provided with tails, on average of 1900 thymine-bases, by terminal transferase. Prior to hybridization, the probe molecules were irradiated with 2500 J/m$^2$ UV-light (254 nm). The hybridization was performed in accordance with "Method III" in the aforementioned instruction manual for the "Gene-Screen" membrane.

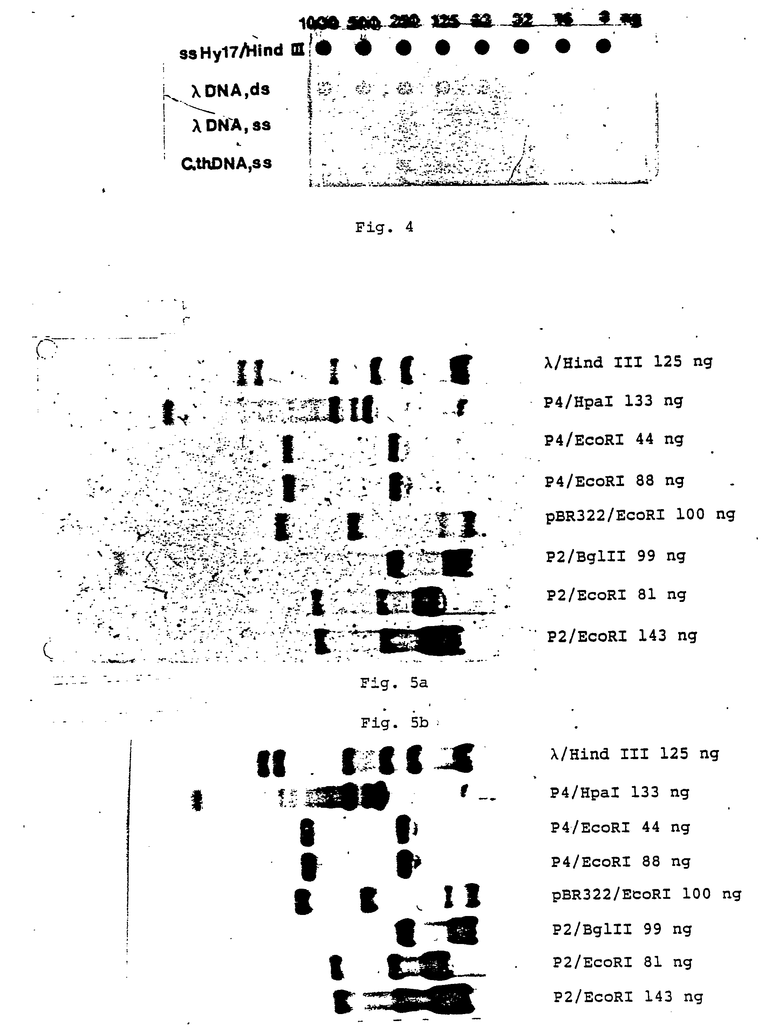

After hybridization, the membrane was washed and incubated with 10% of normal swine serum in tris-buffered saline (TBS=0.02 M Tis-HCl, pH 7.6, with 0.5 M NaCl added) with gentle shaking at room temperature for one hour. This step was followed by incubation for 30 min. at 20°C with a suitable dilution of anti-UV-DNA-antibody in TBS which contained 10% swine serum. After washing (3 × 5 min.) with TBS containing 0.05% of Tween 20, the membrane was incubated for 30 min. with swine-anti-rabbit-antibody which was linked to horseradish-peroxidase (DAKOPATTS A/S, DENMARKO) and diluted 1:200 in TBS with 10% or normal swine serum. The membrane was washed again (3 × 5 min.) and incubated with diaminobenzidine and H$_2$O$_2$ according to Adams, J. C., Histochem Cytochem., *29*: 775 (1981). The results appear in Figure 4.

From Fig. 4 it is clear that only the blots containing Hyl7-DNA, which is homologous to the probe-DNA, are stained. The specificity of the reaction is demonstrated by the absence of a color reaction for ss and ds lambda-DNA, and for C.th-DNA. The stain furthest to the right contains only 8 ng of DNA, and thus provides an indication of the sensitivity of the technique. By increasing the sensitivity at the detection stage, it is believed possible to detect DNA in the pg-range.

Example 3
Photoimmune Detection of DNA
Compared to Ethidium Bromide-Staining

The sensitivity of the present photoimmune assay for detecting DNA was compared to the sensitivity of the conventional ethidium bromide-staining technique according to the following procedure. DNA from P2, P4, lambda and pBR 322 were cleaved with the restriction enzymes HpaI, EcoRI, BglI and HindIII, and separated by agarose-gel electrophoresis (0.7% agarose in the gel; 40 mM tris-acetate buffer with 1 mM EDTA, pH 8). The gel was stained with ethidium bromide (0.5 microgram/ml and visualized over UV-light from a transilluminator (Fotodyne, 300 nm) which simultaneously generated UV-photoproducts. The ethidium bromide-stained gel is shown in Fig. 5a. The DNA in the gel was denatured in 0.2 N NaOH and transferred to a "Gene-Screen" membrane by electroblotting according to the instruction manual for the "Gene-Screen". The membrane was subsequently incubated with swine serum and stained according to the method described in Example 2. The result is shown in Fig. 5b.

A comparison of Figures 5a and 5b reveals that photoimmune detection of DNA according to the present invention (5b) works well, and is more sensitive than conventional ethidium bromide-staining (5a). For instance, in lane 2 from the top, showing P4 DNA fragmented with HpaI, several of the faint bands which can be seen in Fig. 5a are significantly stronger in Fig. 5b, thereby indicating the increased sensitivity of the photoimmune detection over ethidium bromide-staining.

The present invention may be embodied in other specific forms without departing from the spirit or essential attributes thereof, and accordingly, reference should be made to the appended claims, rather than to the foregoing specification, as indicating the scope of the invention.

**Claims**

1. A method for detecting the presence of specific nucleic acid in a sample comprising;
   (a) preparing a polynucleotide probe which will hybridize with the nucleic acid to be detected;
   (b) labelling said probe by inducing formation of UV-photoproducts by exposure to ultraviolet radiation,
   (c) contacting said UV-labelled probe with single-stranded nucleic acid from said sample under hybridizing conditions; and
   (d) detecting hybrid complexes with anti-UV-nucleic acid antibodies labelled with a signalling means.

2. A method according to claim 1 wherein the polynucleotide probe includes a polypyrimidine nucleotide sequence at the 3'-OH terminus.

3. A method according to claim 2 wherein the polypyrimidine nucleotide sequence is generated by cloning said probe directly into a polypyrimidine nucleotide sequence.

4. A method according to claim 2 wherein the polypyrimidine nucleotide sequence is generated by polymerizing pyrimidine nucleotide molecules onto the 3'-OH terminus of said probe.

5. A method according to claim 1 wherein said anti-UV-nucleic acid antibodies are bound to the UV-labelled probe prior to contact with nucleic acid from the sample.

6. A method according to claim 1 wherein the signalling means comprises a color-indicating compound.

7. A method according to claim 1 wherein the signalling means comprises a reagent of high electron density.

8. A method according to claim 1 wherein the signalling means comprises an enzyme that catalyzes a reaction forming a detectable reaction product.

9. A method according to claim 1 wherein a secondary antibody which binds to the anti-UV-nucleic acid antibody is labelled with the signalling means.

10. A method according to claim 9 wherein the signalling means comprise an enzyme that catalyzes a reaction forming a detectable product.

11. A method according to claim 10 wherein the enzyme catalyzes a color-developing reaction.

12. A method according to claim 7 wherein the enzyme is horseradish-peroxidase.

13. A method according to claim 1 wherein the sample nucleic acid is immobilized on a solid support.

14. A method for detecting the presence of specific nucleic acid in a sample comprising:

(a) contacting single-stranded nucleic acid from said sample affixed to a solid support with a polynucleotide probe which will hybridize with the nucleic acid to be detected under hybridizing conditions, said probe including a single-stranded polypyrimidine nucleotide sequence extending from its 3'-OH terminal end irradiated to cause the formation of UV-photoproducts;

(b) incubating the support-bound nucleic acid with anti-UV-nucleic acid antibody to the UV-irradiated probe; and

(c) incubating the support-bound nucleic acid with secondary antibody which binds to said anti-UV-nucleic acid antibody, said secondary antibody being linked to an enzyme that catalyzes a reaction forming a detectable product.

15. A method according to claim 14 wherein said anti-UV-nucleic acid antibodies are bound to the UV-irradiated probe prior to incubation with the support-bound nucleic acid.

16. A kit for quantifying DNA in a test sample comprising:

(a) a supply of anti-UV-DNA antibody;

(b) a panel of DNA-containing samples of known DNA concentrations as standards;

(c) signalling means for indicating the binding of anti-UV-DNA antibody to DNA in the test sample; and

(d) means for comparing the signal level generated by the DNA in said test sample with the signal levels generated by the various members of the standard panel.

17. A kit for detecting the presence of specific nucleic acid in a suspect sample comprising:

(a) a supply of UV-labeled nucleic acid probe molecules containing UV-photoproducts, said probe selected so as to hybridize with the nucleic acid to be detected;

(b) means for contacting the suspect sample with said probe to form hybrid complexes; and

(c) a supply of anti-UV-nucleic acid antibodies which bind said UV-labeled probe, said antibodies having a signalling means associated therewith.

18. A kit according to claim 17 wherein the associated signalling means comprises an enzyme-linked secondary antibody which binds said anti-UV-nucleic acid antibodies.

19. A kit according to claim 18 wherein the probe includes a polypyrimidine nucleotide sequence at the 3'-OH terminus.

**Patentansprüche**

1. Verfahren zum Detektieren der Anwesenheit einer spezifischen Nukleinsäure in einer Probe mit:

a) Zubereiten eines Polynukleotid-Testmittels, welches mit der zu detektierenden Nukleinsäure hybridisiert;

b) Markieren des Testmittels durch das Induzieren der Bildung von UV-Fotoprodukten, indem es ultravioletter Strahlung ausgesetzt wird,

c) Inkontaktbringen des UV-markierten Testmittels mit Einzelstrangnukleinsäure aus der Probe unter Hybridisierungsbedingungen; und

d) Detektieren von Hybridkomplexen mit Anti-UV-Nukleinsäureantikörpen, welche mit einem Anzeigemittel markiert sind.

2. Verfahren nach Anspruch 1, dadurch gekennzeichnet, daß das Polynukleotid-Testmittel eine Polypyrimidin-Nukleotidsequenz an dem 3'-OH-terminalen Ende enthält.

3. Verfahren nach Anspruch 2, dadurch gekennzeichnet, daß die Polypyrimidin-Nukleotidsequenz durch Klonieren des Testmittels direkt in eine Polypyrimidin-Nukleotidsequenz erzeugt wird.

4. Verfahren nach Anspruch 2, dadurch gekennzeichnet, daß die Polypyrimidin-Nukleotidsequenz durch Polymerisieren von Pyrimidin-Nukleotidmolekülen auf dem 3'-OH-terminalen Ende des Testmittels erzeugt wird.

5. Verfahren nach Anspruch 1, dadurch gekennzeichnet, daß die Anti-UV-Nukleinsäureantikörper vor dem Kontakt mit Nukleinsäure aus der Probe an das UV-markierte Testmittel gebunden werden.

6. Verfahren nach Anspruch 1, dadurch gekennzeichnet, daß das Anzeigemittel eine Farbindikatorverbindung ist.

7. Verfahren nach Anspruch 1, dadurch gekennzeichnet, daß das Anzeigemittel ein Reagens mit hoher Elektronendichte ist.

8. Verfahren nach Anspruch 1, dadurch gekennzeichnet, daß das Anzeigemittel ein Enzym aufweist, welches die Reaktion zur Bildung eines detektierbaren Reaktionsproduktes katalysiert.

9. Verfahren nach Anspruch 1, dadurch gekennzeichnet, daß ein sekundärer Antikörper, welcher sich mit dem Anti-UV-Nukleinsäureantikörper verbindet, mit dem Anzeigemittel markiert wird.

10. Verfahren nach Anspruch 9, dadurch gekennzeichnet, daß das Anzeigemittel ein Enzym aufweist, welches eine Reaktion zur Bildung eines detektierbaren Produktes katalysiert.

11. Verfahren nach Anspruch 10, dadurch gekennzeichnet, daß das Enzym eine Farbentwicklungsreaktion katalysiert.

12. Verfahren nach Anspruch 7, dadurch gekennzeichnet, daß das Enzym Meerrettich-Peroxidase ist.

13. Verfahren nach Anspruch 1, dadurch gekennzeichnet, daß die Nukleinsäure der Probe auf einem festen Träger fixiert wird.

14. Verfahren zum Detektieren der Anwesenheit einer spezifischen Nukleinsäure in einer Probe mit:

a) Inkontaktbringen von Einzelstrangnukleinsäure aus der Probe, welche an einem festen Träger fixiert ist, mit einem Polynukleotid-Testmittel, welches mit der zu detektierenden Nukleinsäure unter Hybridisierungsbedingungen hybridisiert, wobei das Testmittel eine Einzelstrang-Polypyrimidin-Nukleotidsequenz enthält, welche sich von ihrem 3'-OH-terminalen Ende erstreckt und bestrahlt ist, um die Bildung von UV-Fotoprodukten zu bewirken;

b) Inkubieren der trägergebundenen Nukleinsäure mit Anti-UV-Nukleinsäureantikörpern zu dem UV-bestrahlen Testmittel; und

c) Inkubieren der trägergebundenen Nukleinsäure mit sekundärem Antikörper, welcher eine Verbindung mit dem Anti-UV-Nukleinsäureantikörper eingeht, wobei der sekundäre Antikörper an ein Enzym gekoppelt ist, welches eine Reaktion zur Ausbildung eines detektierbaren Produktes katalysiert.

15. Verfahren nach Anspruch 14, dadurch gekennzeichnet, daß die Anti-UV-Nukleinsäureantikörper vor dem Inkubieren mit der trägergebundenen Nukleinsäure an das UV-bestrahlte Testmittel gebunden werden.

16. Ausrüstung zur mengenmäßigen Bestimmung von DNA in einer Testprobe mit:

a) einer Zufuhr von Anti-UV-DNA-Antikörper;

b) einer Platte mit DNA enthaltenden Proben einer bekannten DNA Konzentration als Standards bzw. Bezugsrößen;

c) einem Anzeigemittel zum Anzeigen der Bindung von Anti-UV-DNA-Antikörper an DNA in der Testprobe; und

d) einem Mittel zum Vergleichen der Signalstärke, welche durch die DNA in der Testprobe erzeugt wird mit der Signalstärke, welche durch die verschiedenen Einzelproben der Standardplatte erzeugt wird.

17. Ausrüstung zum Detektieren der Anwesenheit einer spezifischen Nukleinsäure in einer verdächtigen Probe mit:

a) einer Zufuhr von UV-markierten Nukleinsäure-Testmittelmolekülen, welche UV-Fotoprodukte enthalten, wobei das Testmittel so ausgewählt ist, daß es mit der zu detektierenden Nukleinsäure hybridiziert;

b) einem Mittel, um die verdächtige Probe mit dem Testmittel zur Bildung von Hybridkomplexen in Kontakt zu bringen; und

c) einer Zufuhr von Anti-UV-Nukleinsäureantikörpern, welche eine Verbindung mit dem UV-markierten Testmittel eingehen, wobei die Antikörper ein Anzeigemittel haben, was mit ihren verknüpft ist.

18. Ausrüstung nach Anspruch 17, dadurch gekennzeichnet, daß das zugehörige Anzeigemittel einen enzymverknüpften sekundären Antikörper enthält, welcher eine Verbindung mit den Anti-UV-Nukleinsäureantikörpern eingeht.

19. Ausrüstung nach Anspruch 18, dadurch gekennzeichnet, daß das Testmittel eine Polypyrimidin-Nukleotidsequenz an dem 3'-OH-terminalen Ende enthält.

**Revendications**

1. Procédé pour déceler la présence d'un acide nucléique spécifique dans un échantillon, ce procédé comprenant:

(a) la préparation d'une sonde de polynucléotide, qui va former un hybride avec l'acide nucléique à déceler;

(b) le marquage de cette sonde par induction de la formation de photoproduits UV, par exposition à un rayonnement ultra-violet (UV);

(c) la mise en contact de ladite sonde, marquée à l'aide de l'ultra-violet, avec de l'acide nucléique monocaténaire provenant dudit échantillon, dans des conditions d'hybridation; et

(d) la détection de complexes hybrides à l'aide d'anticorps anti-acide nucléique UV, marqués par un moyen capable d'émettre un signal.

2. Procédé selon la revendication 1, dans lequel la sonde de polynucléotide comprend une séquence de nucléotides polypyrimidiniques sur le groupe 3'-OH terminal.

3. Procédé selon la revendication 2, dans lequel la séquence des nucléotides polypyrimidiniques est engendrée par clonage de ladite sonde directement dans une séquence de nucléotides polypyrimidiques.

4. Procédé selon la revendication 2, dans lequel la séquence des nucléotides polypyrimidiniques est engendrée par fixation, par polymérisation de molécules de nucléotides pyrimidiniques, sur le groupe 3'-OH terminal de ladite sonde.

5. Procédé selon la revendication 1, dans lequel lesdits anticorps anti-acides nucléiques-UV sont fixés sur la sonde marquée à l'UV avant la mise en contact avec l'acide nucléique de l'échantillon.

6. Procédé selon la revendication 1, dans lequel le moyen capable d'émettre un signal comprend un composé indicateur coloré.

7. Procédé selon la revendication 1, dans lequel le moyen capable d'émettre un signal comprend un réactif à haute densité d'électrons.

8. Procédé selon la revendication 1, dans lequel le moyen capable d'émettre un signal comprend une enzyme, qui catalyse une réaction formant un produit de réaction décelable.

9. Procédé selon la revendication 1, dans lequel un anticorps secondaire, qui se fixe sur l'anticorps anti-acide nucléique UV, est marqué par le moyen de signalisation (capable d'émettre un signal).

10. Procédé selon la revendication 9, dans lequel le moyen capable d'émettre un signal comprend une enzyme qui catalyse une réaction formant un produit décelable.

11. Procédé selon la revendication 10, dans lequel l'enzyme catalyse une réaction de développement de couleur.

12. Procédé selon la revendication 7, dans lequel l'enzyme est de la peroxydase de raifort.

13. Procédé selon la revendication 1, selon lequel l'acide nucléique de l'échantillon est immobilisé sur un support solide.

14. Procédé pour déceler la présence d'un acide nucléique spécifique dans un échantillon, comprenant:

(a) la mise en contact de l'acide nucléique monocaténaire dudit échantillon, fixé sur un support solide, avec une sonde de polynucléotide qui va former un hybride avec l'acide nucléique à déceler, dans des conditions d'hybridation, ladite sonde comprenant une séquence de nucléotides polypyrimidiniques monocaténaires s'étendant depuis son groupe 3'-OH terminal irradiée pour provoquer la formation de photoproduits obtenus à l'aide de l'ultra-violet (UV);

(b) l'incubation de l'acide nucléique, fixé au support, avec de l'anticorps anti-acide nucléique UV pour donner la sonde irradiée à l'UV; et

(c) l'incubation de l'acide nucléique, fixé sur le support, avec un anticorps secondaire qui se fixe sur ledit anticorps anti-acide nucléique-UV, ledit anticorps secondaire étant relié à une enzyme qui catalyse une réaction formant un produit décelable.

15. Procédé selon la revendication 14, dans lequel lesdits anticorps anti-acide nucléique UV sont fixés sur la sonde irradiée à l'ultra-violet avant l'incubation avec l'acide nucléique fixé sur le support.

16. Nécessaire pour quantifier l'ADN dans un échantillon d'essai, ce nécessaire comprenant:

(a) une source d'anticorps anti-ADN UV;

(b) un groupe d'échantillons contenant ADN, à des concentrations connues en ADN, comme étalons;

(c) un moyen capable d'émettre un signal, pour indiquer la fixation de l'anticorps anti-ADN UV sur l'ADN présent dans l'échantillon d'essai; et

(d) un moyen pour comparer le niveau de signal engendré par l'ADN présent dans ledit échantillon d'essai avec les niveau de signaux engendrés par les divers membres du groupe d'étalons.

17. Nécessaire pour déceler la présence d'un acide nucléique spécifique dans un échantillon suspect, ce nécessaire comprenant:

(a) une source de molécules constituant une sonde, marquee a l'UV, pour recherche d'acide nucléique et contenant des photoproduits obtenus à l'aide de l'ultra-violet (UV), la dite sonde étant choisie de façon à former un hybride avec l'acide nucléique à déceler;

(b) un moyen pour effectuer le contact de l'échantillon suspect avec ladite sonde pour former les complexes hybrides; et

(c) une source d'anticorps anti-acide nucléique UV qui se lient à ladite sonde marquée à l'UV, lesdits anticorps comprenant un moyen de signalisation qui leur est associé.

18. Nécessaire selon la revendication 17, dans lequel le moyen de signalisation (capable d'émettre un signal) associé comprend un anticorps secondaire lié à une enzyme, qui fixe lesdits anticorps anti-acide nucléique UV.

19. Nécessaire selon la revendication 18, dans lequel la sonde comprend une séquence de nucléotides polypyrimidiniques sur le groupe 3'-OH terminal.

EP 0 233 177 B1

FIG. 1a

PYRIMIDINE DIMER

FIG. 1b

SATURATED THYMINE PHOTOPRODUCTS

1

EP 0 233 177 B1

probe DNA

restriction enzyme

denaturation

TTP + terminal transferase

UV

hybridization with DNA on membrane

rabbit anti-UV-DNA antibody

swine anti-rabbit-hrp antibody

hrp-substrate
$H_2O_2$ + DAB-Ni/Co

dying of membrane

Fig. 2

2

Fig. 3

pPYA 101/Uncleaved 100.0 ng : 6

pPHR 20/Kpn I ; 343.0 ng : 5

" " /Pst I ; 6.8 ng : 4
" " " ; 68.0 ng : 3

" " " ; 680.0 ng : 2
rDNA from Physarum P.,
CL/Kpn I ; 250.0 ng : 1

EP 0 233 177 B1

Fig. 4

λ/Hind III 125 ng

P4/HpaI 133 ng

P4/EcoRI 44 ng

P4/EcoRI 88 ng

pBR322/EcoRI 100 ng

P2/BglII 99 ng

P2/EcoRI 81 ng

P2/EcoRI 143 ng

Fig. 5a

Fig. 5b

λ/Hind III 125 ng

P4/HpaI 133 ng

P4/EcoRI 44 ng

P4/EcoRI 88 ng

pBR322/EcoRI 100 ng

P2/BglII 99 ng

P2/EcoRI 81 ng

P2/EcoRI 143 ng

4